# EUROPÄISCHE PATENTANMELDUNG

(11) **EP 0 969 017 A1**
(43) Veröffentlichungstag der Anmeldung: **05.01.2000**
(21) Anmeldenummer: 99111763.1
(22) Anmeldetag: 18.06.1999
(51) Int. Cl.: C07K 14/81, A61K 38/57

(54) **Modifizierter C1-Esterase-Inhibitor zur Blockierung der Infektiosität von HIV**

(30) Priorität: 30.06.1998 DE 19829014
(71) Anmelder: Centeon Pharma GmbH, 35002 Marburg (DE)
(72) Erfinder: Gröner, Albrecht, Dr., 64342 Seeheim (DE); Römisch, Jürgen, Dr., 35041 Marburg (DE)

(57) **Zusammenfassung**

Es wird ein modifizierter C1-Esterase-Inhibitor beschrieben, der einerseits an die Oberfläche von HIV, andererseits aber nicht an humane Zellmembranen bindet. Dieser modifizierte C1-Esterase-Inhibitor ist zur Blockierung der Infektiosität von HIV in vivo und in vitro verwendbar.

## Beschreibung

Gegenstand der Erfindung ist ein modifizierter C1-Esterase-Inhibitor, der zur Blockierung der Infektiosität von Humanimmunodefizienzvirus (HIV) in vivo oder in vitro eingesetzt werden kann.

Es ist bekannt, daß die Entfernung von HIV aus biologischen Flüssigkeiten, vor allem aber aus Blut, Blutplasma oder Blutserum eine wichtige Voraussetzung für ihren risikolosen Einsatz für die verschiedensten medizinischen Zwecke ist. Es sind deshalb auch schon zahlreiche Verfahren vorgeschlagen worden, mit denen eine Entfernung von HIV aus biologischen Flüssigkeiten erreicht werden soll. So ist in der internationalen Patentanmeldung WO 97/07674 ein Verfahren vorgeschlagen worden, mit dem HIV aus biologischen Flüssigkeiten entfernt oder inaktiviert werden können, in dem man sie mit bestimmten Ethylenimin-Oligomeren behandelt. Wichtig ist dabei, daß andere Bestandteile des Blutes, insbesondere die zellulären Bestandteile, vor allem die Erythrozyten, durch eine derartige Behandlung nicht beschädigt werden und die Entfernung von HIV in einfacher Weise und kurzer Zeit durchgeführt werden kann, damit genügend große Mengen gereinigten Blutes in einem wirtschaftlich vertretbaren Verfahren gewonnen werden können.

Es ist auch schon vorgeschlagen worden, HIV aus biologischen Flüssigkeiten mittels einer Filtration durch ein mit dem C1-Inhibitor imprägniertes Material adsorptiv zu entfernen.

Der C1-Inhibitor, auch als C1-Esterase-Inhibitor bezeichnet, ist ein im Blut vorhandenes Protein und ist der Hauptinhibitor des klassischen Weges des Komplementsystems und des Kontaktsystems. Der C1-Inhibitor kann die aktivierte Form des Faktors XII und des Kallikreins hemmen (Schapira M. et al., 1985, Complement 2: 111; Davis A.E., 1988, Ann Rev Immunol 6: 595; Sim R.B. et al., 1979, FEBS Lett 97: 111; De Agostini A. et al., 1984, J Clin Invest 73: 1542; Pixley R.A. et al., 1985, J Biol Chem 260: 1723; Schapira M. et al., 1982, J Clin Invest 69: 462; Van der Graaf F. et al., 1983, J Clin Invest 71: 149; Harpel P.C. et al., 1975, J Clin Invest 55: 593). Der C1-Inhibitor reguliert somit die Aktivitäten von zwei Plasmakaskaden, nämlich das Komplementsystem und das Kontaktsystem, durch die biologisch aktive Peptide erzeugt werden. Der C1-Inhibitor ist deshalb auch ein wichtiger Regulator des Entzündungssystems. Außerdem hemmt der C1-Inhibitor den aktivierten Faktor XI (Meijers J.C.M. et al., 1988, Biochemistry 27: 959; Wuillemin W.A. et al., 1995, Blood 85: 1517). Hieraus ergibt sich, daß der C1-Inhibitor als ein Coagulations-Hemmstoff betrachtet werden kann. Auch der Gewebeplasminogen-Aktivator und Plasmin werden in gewissem Ausmaß von dem C1-Inhibitor gehemmt, obwohl das nicht seine Hauptfunktion ist (Harpel P.C. et al., 1975, J Clin Invest 55: 149; Booth N.A. et al., 1987, Blood 69: 1600).

Der C1-Inhibitor wird in erheblichem Ausmaß durch Reinigung aus Plasma gewonnen und für klinische Anwendungen benutzt, insbesondere bei der Behandlung des hereditären Angioödems, einer Erkrankung, die durch einen genetisch bedingten Mangel des C1-Inhibitors hervorgerufen wird. Außerdem ist bereits beschrieben worden, daß durch Verabreichung des C1-Inhibitors bei systemischen Entzündungen [Internationale Patentanmeldung WO 92/22320 (Genentech Inc.)], bei schweren Verbrennungen, Pankreatitis, Knochenmarkstransplantationen, der Zytokin-Therapie und beim Einsatz in extrakorporalen Blutkreisläufen [DE-A-4 227 762 (Behringwerke AG)] gute therapeutische Erfolge erzielt wurden.

Die vollständige genomische und die cDNA, die für den C1-Inhibitor kodiert, ist bereits kloniert worden (Bock S.C. et al., 1986, Biochemistry 25: 4292; Carter P.E. et al., 1988, Eur J Biochem 173: 163). Verschiedene Varianten des rekombinanten C1-Inhibitors mit Aminosäuremutationen in der P1 und den P3 und/oder P5-Positionen des reaktiven Zentrums als auch Varianten, die aus Patienten mit einem hereditären Angioödem isoliert wurden, sind bereits rekombinant hergestellt worden (Eldering E. et al., 1988, J Biol Chem 263: 11776; Eldering E. et al., 1993, J Biol Chem 267: 7013; Eldering E. et al., 1993, J Clin Invest 91: 1035; US-Patent 5,622,930; Davis A.E. et al., 1992, Nature Genetics 1: 354; Eldering E. et al., 1995, J Biol Chem 270: 2579; Verpy et al., 1995, J Clin Invest 95: 350).

Der C1-Inhibitor gehört zu der großen Familie der Serin-Proteinase Inhibitoren, die auch Serpine genannt werden (Travis J. et al., 1983, Ann Rev Biochem 52: 655; Carrel R.W. et al., 1985, Trends Bioch Sci 10: 20). Auf SDS-Polyacrylamidgelen zeigt der C1-Inhibitor ein Molekulargewicht von etwa 105 kD. Seine Plasmakonzentration liegt bei etwa 270 mg/l (Schapira M et al., 1985, Complement 2: 111; Nuijens JH et al., 1989, J Clin Invest 84: 443). Der C1-Inhibitor ist ein Protein, dessen Plasmaspiegel bei unkomplizierten Infektionen und anderen Entzündungen bis zum zweifachen ansteigen kann (Kalter E.S. et al., 1985, J Infect Dis 151: 1019). Die vermehrte Bildung des C1-Inhibitors bei Entzündungen dient wahrscheinlich dem Schutz des Organismus gegen die schädlichen Wirkungen der intravaskulären Aktivierung des Komplementsystems und des Kontaktsystems während der akuten Reaktionen.

Die Serpine reagieren als Inhibitoren durch Bildung bimolekularer Komplexe mit der zu hemmenden Proteinase. Bei diesen Komplexen wird das aktive Zentrum der Proteinase durch das aktive Zentrum des Serpins gebunden und damit inaktiv (Travis J. et al., 1983, Ann Rev Biochem 52: 655). Die Serpine reagieren spezifisch mit bestimmten Proteinasen, wobei diese Spezifität durch die Aminosäuresequenz des reaktiven Zentrums bestimmt wird.

Die vorliegende Erfindung geht nun von der Beobachtung aus, daß die Infektiosität von HIV durch die Verabreichung eines C1-Inhibitors blockiert werden kann.

Bekanntlich besteht der erste Schrift bei einer HIV-Infektion darin, daß Helfer-T-Lymphozyten (T_{H}-Zellen) des Immunsystems, die an ihrer Oberfläche CD4-Rezeptoren tragen, in eine Wechselwirkung mit den Oberflächenproteinen von HIV treten. Antikörper gegen CD4 blockieren in vitro die HIV-Infektion von T_{H}-Zellen. Auch bei einem Überschuß an freiem CD4-Protein geht die Infektionsrate in vitro zurück. Beide Behandlungen blockieren zwar die Infektion, zerstören jedoch nicht das Virus. In ähnlicher Weise läßt sich erfindungsgemäß durch Bindung von HIV an den C1-Inhibitor die direkte Wechselwirkung zwischen den Oberflächenproteinen von HIV und den CD4-Rezeptoren der T_{H}-Zellen unterbinden.

Der humane C1-Inhibitor weist jedoch nicht nur eine große Affinität zu den Hüllproteinen von HIV, sondern auch eine große Affinität zu den Oberflächenproteinen von Lymphozyten auf. Durch den C1-Inhibitor läßt sich nach Art eines Spacers eine brückenartige Verbindung zwischen dem HIV und der T_{H}-Zelle herstellen. Hierdurch läßt sich allerdings überraschenderweise die Infektiosiät von HIV noch nicht vermindern. Das läßt sich jedoch erreichen, wenn ein modifizierter C1-Esterase-Inhibitor eingesetzt wird, der einerseits eine hohe Bindungsaffinität zu den Hüllproteinen von HIV aufweist, andererseits aber nicht an humane Zellmembranen bindet.

Modifizierte C1-Esterase-Inhibitoren stehen aus unterschiedlichen Quellen zur Verfügung. So zeigen die aus dem Blut von Rindern oder anderen Säugetieren gewonnenen C1-Esterase-Inhibitoren keine Affinität zu humanen Zellmembranen, insbesondere nicht zu den CD4-Rezeptoren der humanen T_{H}-Zellen. Ihre Affinität zu den Oberflächenproteinen von HIV (gp120 und gp41) kann jedoch mit der des humanen C1-Esterase-Inhibitors verglichen werden. Ein tierischer C1-Esterase-Inhibitor ist deshalb in der Lage, an die Oberflächenproteine von HIV zu binden, verhindert aber jeden Kontakt von HIV mit den humanen CD4-Rezeptoren, da der C1-Inhibitor an der Oberflächen der T_{H}-Zellen nicht haftet.

Es besteht jedoch auch die Möglichkeit auf gentechnologischem Weg modifizierte Varianten des humanen C1-Inhibitors herzustellen. Die genaue Aminosäuresequenz des humanen C1-Inhibitor und die DNA-Sequenz, die für diesen humanen C1-Inhibitor kodiert, sind von Bock et al., Biochemistry 25: 4292-4301, 1986 und von Davis et al., PNAS 83: 3161-3165, 1986 beschrieben. Biologisch wirksame Varianten des C1-Esterase-Inhibitors sind aus der Internationalen Patentanmeldung WO 91/06650 bekannt. Diese Varianten können durch rekombinante Expressionsverfahren hergestellt werden, wobei die für den C1-Esterase-Inhibitor kodierende DNA spezifische Deletionen, Einfügungen oder Substitutionen von Nukleotiden aufweist.

Grundsätzlich können zur Blockierung der Infektiosität von HIV alle nicht-humanen C1-Esterase-Inhibitoren eingesetzt werden, die nicht an Lymphozyten binden. Dabei handelt es sich entweder um aus natürlichem Material isolierte, tierische C1-Esterase-Inhibitoren oder um rekombinant herstellte, tierische C1-Esterase-Inhibitoren. Außerdem kann ein rekombinant veränderter, humaner C1-Esterase-Inhibitor, dessen Bindungsfähigkeit an die CD4-Rezeptoren der T-Helferzellen aufgehoben ist, die Infektiosität von HIV blockieren. Hierfür sind Modifikationen der die Bindung an den CD4-Rezeptor bewirkenden Aminosäuresequenz des C1-Inhibitors erforderlich, die nach an sich bekannten rekombinanten Methoden durchgeführt werden können und insbesondere in der Internationalen Patentanmeldung WO 92/22320 beschrieben sind.

Die vorstehend genannten nicht-humanen C1-Esterase-Inhibitoren sowie rekombinant veränderte, humane C1-Esterase-Inhibitoren werden zweckmäßigerweise parenteral und in einer für die therapeutische Wirkung ausreichenden Menge verabreicht. Sie werden im allgemeinen in einer physiologischen Kochsalzlösung, einer Ringerschen Lösung oder einem andere für Injektionszwecke geeigneten Trägermaterial dem Patienten zugeführt.

## Patentansprüche

1. Modifizierter C1-Esterase-Inhibitor, dadurch gekennzeichnet, daß er einerseits an die Oberflächenproteine von HIV, andererseits aber nicht an humane Zellmembranen bindet.

2. C1-Esterase-Inhibitor nach Anspruch 1, dadurch gekennzeichnet, daß er tierischen Ursprungs ist.

3. C1-Esterase-Inhibitor nach Anspruch 1, dadurch gekennzeichnet, daß er rekombinanten Ursprungs ist.

4. Verfahren zur Blockierung der Infektiosität von HIV, dadurch gekennzeichnet, daß man sie mit einem modifizierten C1-Esterase-Inhibitor kontaktiert, der einerseits an HIV, andererseits aber nicht an humanen Zellmembranen bindet.

5. Pharmazeutische Zubereitung, dadurch gekennzeichnet, daß sie einen C1-Esterase-Inhibitor nach den Ansprüchen 1 bis 3 in einer therapeutisch wirksamen Menge enthält.

6. Verwendung eines C1-Esterase-Inhibitor der Ansprüche 1 bis 3, dadurch gekennzeichnet, daß er zur Blockierung der Infektiosität von HIV in vivo oder in vitro eingesetzt wird.
